(19) Europäisches Patentamt
European Patent Office
Office européen des brevets

(11) Numéro de publication : **0 315 517 B1**

(12) # FASCICULE DE BREVET EUROPEEN

(45) Date de publication du fascicule du brevet : **15.05.91 Bulletin 91/20**

(51) Int. Cl.[5] : **C07C 51/60**, C07C 53/42, C07C 55/36, C07C 57/66, C07C 61/04, C07C 63/70

(21) Numéro de dépôt : **88402738.4**

(22) Date de dépôt : **02.11.88**

(54) **Procédé de préparation de chlorures d'acides carboxyliques.**

(30) Priorité : **05.11.87 FR 8715344**

(43) Date de publication de la demande : **10.05.89 Bulletin 89/19**

(45) Mention de la délivrance du brevet : **15.05.91 Bulletin 91/20**

(84) Etats contractants désignés : **AT BE CH DE ES FR GB IT LI NL SE**

(56) Documents cités :
**DE-A- 2 247 764**
**US-A- 4 390 708**
**CHEMICAL ABSTRACTS, vol. 74, no. 23, 7 juin 1971, page 402, résumé no. 124729s, Columbus, Ohio, US; E. BOELEMA et al.: "Peculiar behavior of tetrachloroethylene carbonate in the reaction with alcohols"**

(73) Titulaire : **SOCIETE NATIONALE DES POUDRES ET EXPLOSIFS**
**12, quai Henri IV**
**F-75181 Paris Cédex 04 (FR)**

(72) Inventeur : **Chodorge, Jeannine**
**5 rue Germaine**
**F-92160 Antony (FR)**
Inventeur : **Senet, Jean-Pierre**
**79 rue de la Gare Herbeauvilliers-Buthiers**
**F-77760 La Chapelle La Reine (FR)**
Inventeur : **Wooden, Gary**
**7C Résidence de Bel Air**
**F-91540 Mennecy (FR)**

(74) Mandataire : **Pech, Bernard et al**
**SNPE - Service Propriété Industrielle 12, Quai Henri IV**
**F-75181 Paris Cédex 04 (FR)**

EP 0 315 517 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

L'invention a pour objet un nouveau procédé de préparation de chlorures d'acides carboxyliques à partir des acides carboxyliques correspondants.

Différents procédés de préparation de chlorures d'acides carboxyliques à partir d'acides carboxyliques ont été proposés (cf. "Methoden der Organischen Chemie" Houben-Weyl, Vol. 8, pp. 464 et suivantes).

Ainsi on peut utiliser

– le pentachlorure de phosphore

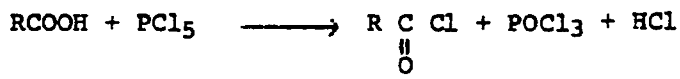

mais c'est un produit solide et sensible à l'hydrolyse par conséquent peu commode d'emploi,

– le trichlorure de phosphore,

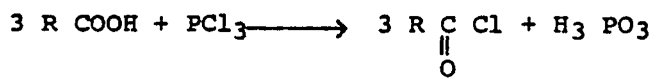

un grand excès de l'ordre de 25 à 100% est nécessaire et les rendements sont très variables en raison de nombreuses réactions secondaires ("The Chemistry of Acyl halides" Saul Pataï, Inter-sciences Publishers - Wiley & sons (1972) p. 40-41).

– le chlorure de thionyle,

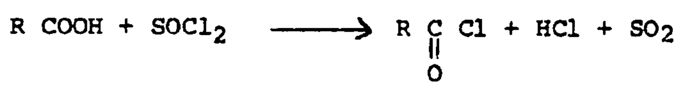

l'addition d'un catalyseur est généralement nécessaire (H.M. Bosshard et al. Helv. Chem. Acta (1959) 62 1633)

– le chlorure d'oxalyle,

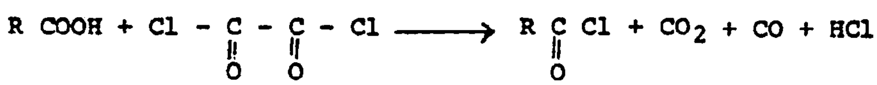

sa toxicité est élevée et c'est un produit cher (N.O.V. Sonntag et al. J. Am. Oil Chemist Soc. (1954) 31, 151)

– le phosgène

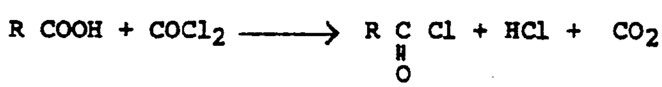

mais en l'absence de catalyseur la réaction doit être effectuée à haute température et sous forte pression (brevet US 2 657 233). De plus c'est un produit toxique qui nécessite des installations très spécifiques et situées à proximité de son lieu de production car son transport est dangereux.

On constate que l'utilisation des différents procédés selon l'art antérieur présente toujours des inconvénients. Ou bien les chlorures d'acides sont obtenus avec des sous-produits gênants ou bien la réaction est difficile à mettre en oeuvre en raison des problèmes de toxicité.

Il était par conséquent très important de disposer d'un nouveau procédé de préparation des chlorures d'acides carboxyliques dans lequel des produits moins dangereux seraient utilisés et qui permettrait d'obtenir des chlorures d'acides de grande pureté.

La présente invention répond à cette attente et a pour objet un procédé de préparation des chlorures d'acides avec un réactif peu toxique et non générateur d'impuretés.

Le procédé selon l'invention est un procédé de préparation des chlorures d'acides carboxyliques par réaction du carbonate de tétrachloroéthylène sur les acides carboxyliques correspondants, à une température

comprise entre 80°C et 160°C.

Le schéma réactionnel est le suivant :

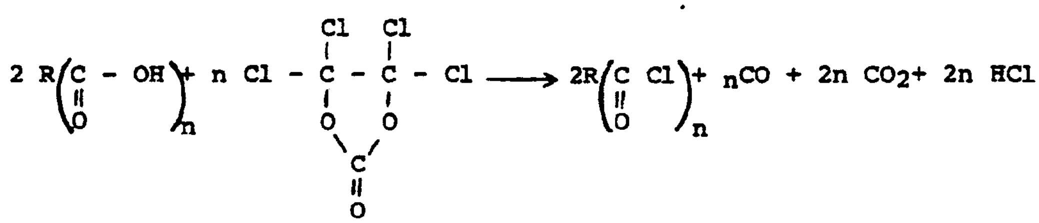

Le carbonate de tétrachloroéthylène utile pour la réalisation du procédé selon l'invention est un composé connu. Il peut être préparé facilement par réaction photochimique du chlore sur le carbonate d'éthylène comme décrit dans le brevet US 2 816 287 :

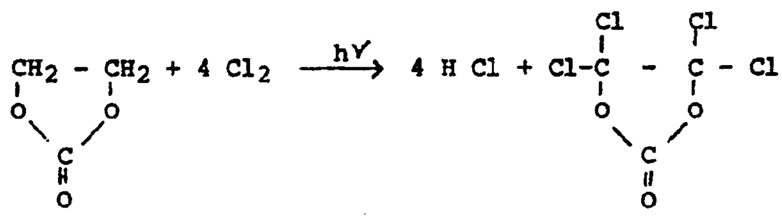

Les acides carboxyliques sur lesquels il est mis à réagir sont très nombreux et variés. On les trouve dans le commerce ou on les prépare selon des méthodes connues.

L'addition d'un catalyseur n'est pas nécessaire.

Un solvant pourra être utilisé lorsque cela est vraiment indispensable comme par exemple lorsque les chlorures d'acide ont un faible point d'ébullition ou lorsque les acides ont un point de fusion élevé.

Les chlorures d'acides obtenus sont par conséquent très purs, exempts de produits parasites.

La mise en oeuvre du procédé est simple. Une distillation n'est pas indispensable pour récupérer les chlorures d'acides et cet avantage est particulièrement apprécié pour la préparation des chlorures d'acides fragiles ou à point d'ébullition très élevé.

Ci-après l'invention est décrite de manière détaillée.

Le procédé selon l'invention s'applique en particulier aux acides carboxyliques de formule R $(COOH)_n$ dans laquelle **R** représente

– un radical alkyle linéaire ou ramifié en $C_1$ à $C_{30}$ qui peut contenir une ou plusieurs double ou triple liaisons carbone-carbone et un ou plusieurs substituants choisis parmi les atomes d'halogène, les radicaux aryles, aryloxy, alcoxy, alkyloxycarbonyles, aryloxycarbonyles et hétérocycliques à 5 ou 6 chaînons saturés ou insaturés, le ou les hétéroatome(s) étant choisis parmi les atomes de soufre ou d'oxygène,

– un radical cycloalkyle en $C_3$ à $C_7$ qui peut comporter une ou plusieurs double liaison carbone-carbone lorsqu'il a de 5 à 7 atomes de carbone, et qui peut porter un ou plusieurs substituants choisis parmi les atomes d'halogène, les radicaux alkyles, alkényles, aryles, alkyloxycarbonyles et aryloxycarbonyles,

– un radical aromatique ou hétéroaromatique condensé ou non, le ou les hétéroatome(s) étant choisis parmi les atomes de soufre, d'oxygène ou d'azote, qui peut porter un ou plusieurs substituants choisis parmi les atomes d'halogène les radicaux alkyles, halogénoalkyles, comportant ou non une ou plusieurs double ou triple liaisons, les radicaux aryles, alcoxy, aryloxy, alkyloxycarbonyles et aryloxycarbonyles et les groupes nitro et cyano,

– un radical hétérocyclique non aromatique à 5 ou 6 chaînons, comportant un ou plusieurs hétéroatomes choisis parmi l'oxygène ou le soufre, pouvant comporter une ou plusieurs doubles liaisons et substitué ou non par un ou plusieurs radicaux alkyles, alkyloxycarbonyles et aryloxycarbonyles,

et n représente un nombre entier pouvant prendre les valeurs 1 à 4.

Plus particulièrement le radical R représente

– un radical alkyle linéaire ou ramifié en $C_1$ à $C_{20}$, qui peut contenir une ou plusieurs double ou triple liaisons carbone-carbone et un ou plusieurs substituants choisis parmi les atomes de chlore, brome ou fluor, les

radicaux alcoxy en $C_1$ à $C_4$, phényle, naphtyle, phénoxy, naphtoxy, alkyloxycarbonyles en $C_1$ à $C_4$, phénoxycarbonyle, furyle, pyrannyle et thiényle,

– un radical cycloalkyle en $C_3$ à $C_6$ qui peut comporter une double liaison lorsqu'il a de 5 à 6 atomes de carbone et qui peut porter un ou plusieurs substituants choisis parmi les radicaux alkyles et alkényles en $C_1$ à $C_5$,

– un radical aromatique ou hétéroaromatique, le ou les hétéroatomes étant choisis parmi les atomes de soufre, d'oxygène ou d'azote, comportant des cycles condensés ou non à 5 ou 6 chaînons et qui peut porter un ou plusieurs substituants choisis parmi les atomes d'halogène, les radicaux alkyles, fluoro- ou chloroalkyles en $C_1$ à $C_{12}$, comportant ou non une ou plusieurs double ou triple liaisons, les radicaux aryles en $C_6$ à $C_{10}$, alcoxy en $C_1$ à $C_{12}$, aryloxy en $C_6$ à $C_{10}$, alkyloxycarbonyles en $C_1$ à $C_4$ et phénoxycarbonyle, les groupes nitro et cyano, en particulier le radical phényl, naphtyl, furyl, benzofuryle, phénoxy et naphtoxy, et n représente la valeur 1 ou 2.

Comme exemple d'acides qui peuvent être utilisés dans le cadre de l'invention on peut citer l'acide propionique, butyrique, isobutyrique, valérique, isovalérique, pivalique, hexanoïque, octanoïque, décanoïque, laurique, palmitique, stéarique, triacontanoïque, undécylénique, acrylique, méthacrylique, crotonique, isocrotonique, propargylique, vinylacétique, oléique, chloroacétique, dichloroacétique, trichloroacétique, bromoacétique, dibromoacétique, tribromoacétique, alpha-chloropropionique, alpha-chlorobutyrique, malonique, succinique, adipique, sébacique, le monométhylester de l'acide malonique, le monométhylester de l'acide succinique, l'acide phénylacétique, phényloxyacétique, bêta-phénylpropionique, bêta-phényloxypropionique, cyclopropane-carboxylique, diméthyl-2,2 (méthyl-2 propényle)-3 cyclopropane-carboxylique, cyclohexane-carboxylique, cyclohexane-dicarboxylique-1,4, cyclohexène-carboxylique, thiényle-2 butyrique-4, méthoxyacétique, tiglique, angélique, fumarique, maléïque, brassylique, benzoïque, toluique, les acides halogénobenzoïques, les acides mononitrobenzoïques, les acides cyanobenzoïques, l'acide isophtalique, téréphtalique, phénoxy-3 benzoïque, tert-butyl-4 benzoïque, furoïque, benzofuroïque, thénoïque, nicotinique, isonicotinique, quinoleïque, les acides indole-carboxyliques, l'acide biphénylcarboxylique, 3,4,5-méthoxybenzoïque, 4-méthoxycarbonylbenzoïque, les acides trifluorométhylbenzoïques, les acides naphtalènecarboxyliques.

Le carbonate de tétrachloroéthylène est de préférence ajouté progressivement au milieu réactionnel qui a été chauffé et maintenu à la température choisie. On l'utilise généralement en une quantité comprise entre 0,50 et 0,75 équivalent par équivalent de fonction acide à transformer, de préférence entre 0,52 et 0,6 équivalent.

Dans la mesure du possible on préfère réaliser la réaction sans solvant. Lorsque l'on souhaite préparer des chlorures d'acides à faible point d'ébullition ou dont les acides correspondants sont à point de fusion élevé, par exemple supérieur à 140°C, on peut utiliser un ou plusieurs solvants inertes choisis par exemple parmi les solvants aliphatiques chlorés tel que le dichloro-1,2 éthane ou les solvants aromatiques tel que le toluène, le xylène, le chlorobenzène, les dichlorobenzènes.

Bien qu'un catalyseur ne soit absolument pas nécessaire pour effectuer la réaction, l'addition de composés catalytiques peut permettre de réduire le temps des réactions et/ou d'abaisser la température.

Les catalyseurs sont choisis parmi les catalyseurs déjà connus et utilisés dans les réactions de préparation des chlorures d'acides carboxyliques décrites précédemment.

En particulier, ils sont choisis dans le groupe qui comprend :

– les amines tertiaires et leurs chlorhydrates, en particulier la pyridine et la N,N-diméthyl-4 pyridine (DMAP),

– les sels d'ammonium quaternaire et en particulier les chlorures et bromures, par exemple ceux de tétra n-butyle et ceux de benzyle trin-butyle,

– les sels de phosphonium quaternaire,

– les amides N,N disubstitués en particulier le N,N-diméthylformamide et leurs produits de réaction avec les composés tels que $PCl_3$, $PCl_5$, $POCl_3$, $COCl_2$, $SOCl_2$, $(CO)_2Cl_2$ (sels de Vilsmeier-Haack),

– les urées tétrasubstituées, en particulier la tétraméthylurée et la tétrabutylurée, et leurs produits de réaction avec les agents composés tels que le $PCl_3$, $PCl_5$, $POCl_3$, $COCl_2$, $SOCl_2$, $(CO)_2Cl_2$,

– les hexaalkylphosphoramides tel que l'hexaméthylphosphoro triamide (HMPT),

– les sels, en particulier les chlorures, d'hexaalkylguanidinium et leurs chlorhydrates, comme décrit dans la demande de brevet EP 213 976, en particulier les chlorures d'hexaméthyl- et d'hexabutylguanidinium.

La quantité de catalyseur ajouté est généralement comprise entre $10^{-4}$ et $10^{-1}$ équivalent par équivalent de fonction acide à transformer.

La température de la chloruration est comprise entre 80° et 160°C. En l'absence d'un catalyseur on effectue généralement la réaction à une température comprise entre 110° et 160°C, de préférence entre 120°C et 160°C. Lorsqu'un catalyseur est utilisé la réaction est effectuée généralement entre 80° et 150°C et de préférence entre

100° et 135°C.

La durée de la réaction est généralement comprise entre 1 et 4 heures.

Grâce au procédé selon l'invention on obtient de façon simple, à partir de matières premières peu coûteuses et non toxiques des chlorures d'acides d'une grande pureté qui pourront être utilisés sans purification ultérieure.

Les chlorures d'acides sont des composés très connus dont les applications sont nombreuses. Ils sont par exemple utilisés comme intermédiaires de synthèse pour la préparation d'esters, d'amides, de peresters et de peroxydes, de composés polymères, de produits pesticides et pharmaceutiques et dans l'industrie papetière.

Les exemples qui suivent illustrent l'invention sans toutefois la limiter.

Exemples 1 à 11

Mode opératoire général

Dans un réacteur équipé d'une agitation, d'un thermomètre et d'un réfrigérant à reflux, on introduit l'acide carboxylique, le solvant s'il y a lieu, et le catalyseur. On chauffe le mélange réactionnel à la température indiquée et on introduit lentement 0,50 à 0,6 équivalent de carbonate de tétrachloroéthylène (CTCE) par fonction acide à transformer en maintenant la température. A la fin de la réaction (suivie par IR ou CPV), le chlorure d'acide est si nécessaire séparé du carbonate de tétrachloroéthylène résiduel par distillation.

Les conditions opératoires et les résultats sont rassemblés dans le tableau suivant.

TABLEAU 1

| Ex. | Acide | CTCF Qté | Catalyseur | Solvant | T(°C)/ Durée | Rendement (Distillé) |
|---|---|---|---|---|---|---|
| 1 | Et<br>Bu-CH $CO_2$ H<br>144 g (1,00 mole) | 135,6 g<br>0,60 mole | $(Bu_2N)_3{}^{\oplus}C\ Cl^{\ominus}$<br>0,086 g (0,0002 mole) | néant | 120-135°/3 h | 94 % |
| 2 | 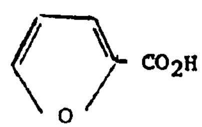<br>33,6 g (0,30 mole) | 37,3 g<br>0,17 mole | 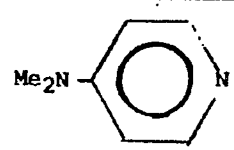<br>0,16 g (0,0013 mole) | $PhCH_3$<br>41 ml | 105°/1 h | 90 % |
| 3 | $PhCO_2H$<br>36,6 g (0,30 mole) | 40,7 g<br>0,18 mole | $(Bu_2N)_2$-C = O<br>0,43 g (0,0015 mole) | néant | 130-135°/1 h | 98 % |
| 4 | $CH_3(CH_2)_{16}\ CO_2H$<br>0,20 mole | 0,11 mole | $(Bu_2N)_3\ C^{\oplus}\ HCl_2{}^{\ominus}$<br>(0,00004 mole) | néant | 105°/1 h | quantitatif (non distillé) |

TABLEAU 1 (Suite)

| Ex. | Acide | CTCE Qté | Catalyseur | Solvant | T(°C)/ Durée | Rendement (Distillé) |
|---|---|---|---|---|---|---|
| 5 | Et<br>$BuCHCO_2H$<br>0,10 mole | 0,06 mole | néant | néant | 130-140°/2 h | 86 % |
| 6 | 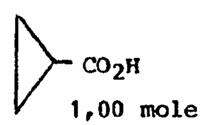<br>1,00 mole | 0,55 mole | néant | néant | 150°/3 h | 83 % |
| 7 | $CH_2=CH(CH_2)_8CO_2H$<br>0,50 mole | 0,28 mole | néant | néant | 150°/2 h | 86 % |
| 8 | $HO_2C(CH_2)_4CO_2H$<br>1,00 mole | 1,05 mole | néant | néant | 150°/3 h | 60 % |
| 9 | 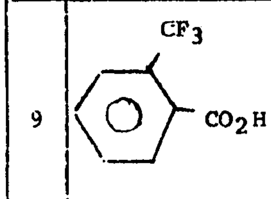<br>0,10 mole | 0,055 mole | néant | néant | 120°/1 h | 91 % |

TABLEAU 1 (Suite)

| Ex. | Acide | CTCE Qté | Catalyseur | Solvant | T(°C)/Durée | Rendement* |
|---|---|---|---|---|---|---|
| 10 | 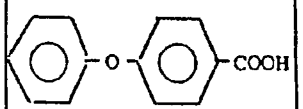 64,2 g (0,3 mole) | 35,9 g 0,159 mole | néant | PhCl 150 g | 120-132°/12h | 62 % |
| 11 | 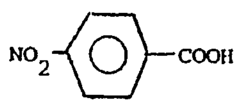 50,1 g (0,3 mole) | 35,9 g 0,159 mole | néant | O-di chloro benzène 50 g | 140-160°/10 h | 77 % |

* Rendement déterminé à partir du taux de chlorure d'acide

**Revendications**

1. Procédé de préparation de chlorures d'acides carboxyliques caractérisé en ce que l'on fait réagir sur un acide carboxylique le carbonate de tétrachloroéthylène à une température comprise entre 80°C et 160°C.

2. Procédé selon la revendication 1 caractérisé en ce que le carbonate de tétrachloroéthylène est ajouté progressivement au milieu réactionnel préalablement chauffé.

3. Procédé selon la revendication 1 ou 2 caractérisé en ce que le carbonate de tétrachloroéthylène est ajouté en quantité comprise entre 0,50 et 0,75 équivalent par équivalent de fonction acide à transformer, de préférence entre 0,52 et 0,6 équivalent.

4. Procédé selon l'une quelconque des revendications précédentes caractérisé en ce que l'acide carboxylique a la formule $R(COOH)_n$ dans laquelle R représente

- un radical alkyle linéaire ou ramifié en $C_1$ à $C_{30}$ qui peut contenir une ou plusieurs double ou triple liaisons carbone-carbone et un ou plusieurs substituants choisis parmi les atomes d'halogène, les radicaux aryles, aryloxy, alcoxy, alkyloxycarbonyles, aryloxycarbonyles et hétérocycliques à 5 ou 6 chaînons saturés ou insaturés, le ou les hétéroatome(s) étant choisis parmi les atomes de soufre ou d'oxygène,
- un radical cycloalkyle en $C_3$ à $C_7$ qui peut comporter une ou plusieurs double liaison carbone-carbone lorsqu'il a de 5 à 7 atomes de carbone, et qui peut porter un ou plusieurs substituants choisis parmi les atomes d'halogène, les radicaux alkyles, alkényles, aryles, alkyloxycarbonyles et aryloxycarbonyles,
- un radical aromatique ou hétéroaromatique condensé ou non, le ou les hétéroatome(s) étant choisis parmi les atomes de soufre, d'oxygène ou d'azote, qui peut porter un ou plusieurs substituants choisis parmi les atomes d'halogène les radicaux alkyles, halogénoalkyles, comportant ou non une ou plusieurs double ou triple liaisons, les radicaux aryles, alcoxy, aryloxy, alkyloxycarbonyles et aryloxycarbonyles et les groupes nitro et cyano,
- un radical hétérocyclique non aromatique à 5 ou 6 chaînons, comportant un ou plusieurs hétéroatomes choisis parmi l'oxygène ou le soufre, pouvant comporter une ou plusieurs double liaisons et substitué ou non par un ou plusieurs radicaux alkyles, alkyloxycarbonyles et aryloxycarbonyles,

et n représente un nombre entier pouvant prendre les valeurs 1 à 4.

5. Procédé selon la revendication 4 caractérisé en ce que le radical R représente

- un radical alkyle linéaire ou ramifié en $C_1$ à $C_{20}$, qui peut contenir une ou plusieurs double ou triple liaisons carbone-carbone et un ou plusieurs substituants choisis parmi les atomes de chlore, brome ou fluor, les radicaux alcoxy en $C_1$ à $C_4$, phényle, naphtyle, phénoxy, naphtoxy, alkyloxycarbonyles en $C_1$ à $C_4$, phénoxycarbonyle, furyle, pyrannyle et thiényle,
- un radical cycloalkyle en $C_3$ à $C_6$ qui peut comporter une double liaison lorsqu'il a de 5 à 6 atomes de carbone et qui peut porter un ou plusieurs substituants choisis parmi les radicaux alkyles et alkényles en $C_1$ à $C_5$,
- un radical aromatique ou hétéroaromatique, le ou les hétéroatomes étant choisis parmi les atomes de soufre, d'oxygène ou d'azote, comportant des cycles condensés ou non à 5 ou 6 chaînons et qui peut porter un ou plusieurs substituants choisis parmi les atomes d'halogène, les radicaux alkyles, fluoro- ou chloroalkyles en $C_1$ à $C_{12}$, comportant ou non une ou plusieurs double ou triple liaisons, les radicaux aryles en $C_6$ à $C_{10}$, alcoxy en $C_1$ à $C_{12}$, aryloxy en $C_6$ à $C_{10}$, alkyloxycarbonyles en $C_1$ à $C_4$ et phénoxycarbonyle, les groupes nitro et cyano,

et n représente la valeur 1 ou 2.

6. Procédé selon l'une quelconque des revendications précédentes caractérisé en ce qu'on utilise un ou plusieurs solvants choisis parmi les solvants aliphatiques chlorés et les solvants aromatiques.

7. Procédé selon l'une quelconque des revendications précédentes caractérisé en ce qu'on utilise un catalyseur choisi dans le groupe qui comprend :

- les amines tertiaires et leurs chlorhydrates,
- les sels d'ammonium quaternaire,
- les sels de phosphonium quaternaire,
- les amides N,N disubstituées, et leurs produits de réaction avec $PCl_3$, $PCl_5$, $POCl_3$, $COCl_2$, $SOCl_2$, $(CO)_2Cl_2$,
- les urées tétrasubstituées et leurs produits de réaction avec $PCl_3$, $PCl_5$, $POCl_3$, $COCl_2$, $SOCl_2$, $(CO)_2Cl_2$,
- les hexaalkylphosphoramides,
- les sels d'hexaalkylguanidinium et leurs chlorhydrates.

8. Procédé selon la revendication précédente caractérisé en ce que le catalyseur est utilisé en quantité comprise entre $10^{-4}$ et $10^{-1}$ équivalent par équivalent de fonction acide à transformer.

9. Procédé selon l'une quelconque des revendications précédentes caractérisé en ce que la température est comprise entre 110 et 160°C en l'absence de catalyseur et entre 80 et 150°C lorsqu'un catalyseur est utilisé.

10. Procédé selon la revendication précédente caractérisé en ce que la température est comprise entre 120° et 160°C en l'absence de catalyseur et entre 100° et 135°C lorsqu'un catalyseur est utilisé.

## Claims

1. Process for the preparation of carboxylic acid chlorides characterised in that tetrachloroethylene carbonate is reacted with a carboxylic acid at a temperature between 80°C and 160°C.

2. Process according to Claim 1, characterised in that the tetrachloroethylene carbonate is added progressively to the preheated reaction medium.

3. Process according to Claim 1 or 2, characterised in that the tetrachloroethylene carbonate is added in a quantity of between 0.50 and 0.75 equivalents per equivalent of acidic functional group to be converted, preferably between 0.52 and 0.6 equivalents.

4. Process according to any one of the preceding claims, characterised in that the carboxylic acid has the formula $R(COOH)_n$, in which R denotes

– a linear or branched $C_1$-$C_{30}$ alkyl radical which may contain one or more double or triple carbon-carbon bonds and one or more substituents chosen from halogens atoms and aryl, aryloxy, alkoxy, alkoxycarbonyl and aryloxycarbonyl radicals and heterocyclic radicals containing 5 or 6 saturated or unsaturated chain links, the hetero atom(s) being chosen from sulphur and oxygen atoms,

– a $C_3$-$C_7$ cycloalkyl radical which may contain one or more double carbon-carbon bonds when it contains 5 to 7 carbon atoms, and which may carry one or more substituents chosen from halogen atoms and alkyl, alkenyl, aryl, alkoxycarbonyl and aryloxycarbonyl radicals,

– an aromatic or heteroaromatic radical, condensed or otherwise, the heteroatom(s) being chosen from sulphur, oxygen and nitrogen atoms, which radical may carry one or more substituents chosen from halogen atoms, alkyl or haloalkyl radicals containing or not containing one or more double or triple bonds, aryl, alkoxy, aryloxy, alkoxycarbonyl and aryloxycarbonyl radicals and nitro and cyano groups,

– a nonaromatic heterocyclic radical containing 5 or 6 chain links, containing one or more heteroatoms chosen from oxygen and sulphur, which may contain one or more double bonds and is substituted or otherwise by one or more alkyl, alkoxycarbonyl and aryloxycarbonyl radicals, and n denotes an integer which may assume the values 1 to 4.

5. Process according to Claim 4, characterised in that the radical R denotes

– a linear or branched $C_1$-$C_{20}$ alkyl radical which may contain one or more double or triple carboncarbon bonds and one or more substituents chosen from chlorine, bromine and fluorine atoms and $C_1$-$C_4$ alkoxy, phenyl, naphthyl, phenoxy, naphthoxy, $C_1$-$C_4$ alkoxycarbonyl, phenoxycarbonyl, furyl, pyranyl, and thienyl radicals,

– a $C_3$-$C_6$ cycloalkyl radical which may contain a double bond when it has from 5 to 6 carbon atoms and which may carry one or more substituents chosen from $C_1$-$C_5$ alkyl and alkenyl radicals,

– an aromatic or heteroaromatic radical, the heteroatom(s) being chosen from sulphur, oxygen and nitrogen atoms, containing condensed or uncondensed rings containing 5 or 6 chain links and which may carry one or more substituents chosen from halogen atoms and $C_1$-$C_{12}$ alkyl, fluoro- or chloroalkyl radicals containing or not containing one or more double or triple bonds, $C_6$-$C_{10}$ aryl, $C_1$-$C_{12}$ alkoxy, $C_6$-$C_{10}$ aryloxy, $C_1$-$C_4$ alkoxycarbonyl and phenoxycarbonyl radicals and nitro and cyano groups,

and n denotes the value 1 or 2.

6. Process according to any one of the preceding claims, characterised in that one or more solvents chosen from chlorinated aliphatic solvents and aromatic solvents is (are) employed.

7. Process according to any one of the preceding claims, characterised in that a catalyst is employed, which is chosen from the group which comprises :

– tertiary amines and their hydrochlorides,

– quaternary ammonium salts,

– quaternary phosphonium salts,

– N, N-disubstituted amides and their reaction products with $PCl_3$, $PCl_5$, $POCl_3$, $COCl_2$, $SOCl_2$ $(CO)_2Cl_2$,

– tetrasubstituted ureas and their reaction products with $PCl_3$, $PCl_5$, $POCl_3$, $COCl_2$, $SOCl_2$ and $(CO)_2Cl_2$,

– hexaalkylphosphoramides,

– hexaalkylguanidium salts and their hydrochlorides.

8. Process according to the preceding claim, characterised in that the catalyst is employed in a quantity of between $10^{-4}$ and $10^{-1}$ equivalents per equivalent of acidic functional group to be converted.

9. Process according to any one of the preceding claims, characterised in that the temperature is between 110 and 160°C in the absence of catalyst and between 80 and 150°C when a catalyst is employed.

10. Process according to the preceding claim, characterised in that the temperature is between 120° and 160°C in the absence of catalyst and between 100° and 135°C when a catalyst is employed.

**Ansprüche**

1.Verfahren zur Herstellung von Carbonsäurechloriden, dadurch gekennzeichnet, daß eine Carbonsäure bei einer Temperatur von 80 bis 160°C mit Tetrachlorethylencarbonat umgesetzt wird.

2. Verfahren nach Anspruch 1, dadurch gekennzeichnet, daß das Tetrachlorethylencarbonat dem zuvor erhitzten Reaktionsmedium fortschreitend zugesetzt wird.

3. Verfahren nach Anspruch 1 oder 2, dadurch gekennzeichnet, daß das Tetrachlorethylencarbonat in einer Menge von 0,5 bis 0,75 Äquivalent pro Äquivalent umzuwandelnder Säurefunktion zugesetzt wird.

4. Verfahren nach einem der vorhergehenden Ansprüche, dadurch gekennzeichnet, daß die Carbonsäure die Formel $R(COOH)_n$ besitzt, in der bedeuten :

Die Gruppe R

– eine geradkettige oder Vereweigte $C_{1-30}$-Alkylgruppe, die eine oder mehrere Kohlenstoff-Kolhenstoff-Doppelbindungen oder -Dreifachbindungen und einen oder mehrere Substituenten enthalten kann, die ausgewählt sind unter Halogenatomen, Arylgruppen Aryloxygruppen, Alkoxygruppen, Alkyloxycarbonylgruppen, Aryloxycarbonylgruppen und gesättigten oder ungesättigten heterocyclischen Resten mit fünf oder sechs Ringgliedern, wobei das oder die Heteroatome unter Schwefelund Sauerstoffatomen ausgewählt sind,

– eine $C_{3-7}$-Cycloalkylgruppe, die eine oder mehrere Kolhenstoff-Kolhenstoff-Doppelbindungen enthalten kann, sofern sie fünf bis sieben Kolhenstoffatome aufweist und einen oder mehrere unter Halogenatomen, Alkylgruppen Alkenylgruppen, Arylgruppen, Alkyloxycarbonylgruppen und Aryloxycarbonylgruppen ausgewählten Substituenten tragen kann,

– eine kondensierte oder nichtkondensierte aromatische oder heteroaromatische Gruppe, wobei das oder die Heteroatome unter Schwefelatomen, Sauerstoffatomen und Stickstoffatomen ausgewählt sind, die einen oder mehrere unter Halogenatomen, Alkylgruppen, Halogenalkylgruppen, die gegebenenfalls eine oder mehrere Doppel- oder Dreifachbindungen aufweisen, Arylgruppen, Alkoxygruppen, Aryloxygruppen, Alkyloxycarbonylgruppen und Aryloxycarbonylgruppen sowie Nitro und Cyano ausgewählte Substituenten tragen kann,

– eine nichtaromatische heterocyclische Gruppe mit fünf oder sechs Ringgliedern, die ein oder mehrere unter Sauerstoff und Schwefel ausgewählte Heteroatome enthält, eine oder mehrere Doppelbindungen enthalten kann und gegebenenfalls mit einer oder mehreren Alkylgruppen, Alkoxycarbonylgruppen- und Aryloxycarbonylgruppen substituiert ist, n eine ganze Zahl, die Werte von 1 bis 4 annehmen kann.

5. Verfahren nach Anspruch 4, dadurch gekennzeichnet, daß bedeuten :

Die Gruppe R

– eine geradkettige oder verzweigte $C_{1-20}$ -Alkylgruppe, die eine oder mehrere Kohlenstoff-Kohlenstoff-Doppeloder Dreifachbindungen und einen oder mehrere Substituenten enthalten kann, die ausgewählt sind unter Chlor-, Brom- und Fluoratomen, $C_{1-4}$ -Alkoxygruppen, Phenyl, Naphthyl, Phenoxy, Naphthoxy, $C_{1-4}$ Alkyloxycarbonylgruppen, Phenoxycarbonyl, Furyl, Pyranyl und Thienyl,

– eine $C_{3-6}$-Cycloalkylgruppe, die eine Doppelbindung enthalten kann, sofern sie fünf bis sechs Kohlenstoffatome besitzt, und einen oder mehrere unter $C_{1-5}$ -Alkylgruppen und $C_{1-5}$ -Alkeenylgruppen ausgewählte Substituenten tragen kann,

– eine aromatische oder heteroaromatische Gruppe, wobei das oder die Heteroatome unter Schwefel-, Sauerstoffund Stickstoffatomen ausgewählt sind, mit kondensierten oder nichtkondensierten Ringen mit fünf oder sechs Ringgliedern,und die einen oder mehrere Substituenten tragen kann, die ausgewählt sind unter Halogenatomen, $C_{1-12}$ -Alkylgruppen, $C_{1-12}$ -Fluoralkylgruppen und $C_{1-12}$ -Chloralkylgruppen, die gegebenenfalls eine oder mehrere Doppel- oder Dreifachbindungen aufweisen, $C_{6-10}$ -Arylgruppen, $C_{1-12}$ - Alkoxygruppen, $C_{6-10}$ -Aryloxygruppen, $C_{1-4}$ -Alkyloxycarbonylgruppen und Phenoxycarbonyl, Nitro und Cyano,

und

n den Wert 1 oder 2

6. Verfahren nach einem der vorhergehenden Ansprüche, dadurch gekennzeichnet, daß ein oder mehrere unter chlorierten aliphatischen und aromatischen Lösungsmitteln ausgewählte Lösungsmittel verwendet werden.

7. Verfahren nach einem der vorhergehenden Ansprüche, dadurch gekennzeichnet, daß ein Katalysator verwendet wird, der ausgewählt ist unter

- tertiären Aminen und ihren Hydrochloriden,
- quaternären Ammoniumsalzen,
- quaternären Phosphoniumsalzen,
- N,N-disubstituierten Amiden und ihren Reaktionsprodukten mit $PCl_3$, $PCl_5$, $POCl_3$, $COCl_2$, $SOCl_2$ und $(CO)_2Cl_2$,
- tetrasubstituierten Harnstoffen und ihren Reaktionsprodukten mit $PCl_3$, $PCl_5$, $POCl_3$, $COCl_2$, $SOCl_2$ und $(CO)_2Cl_2$,
- Hexaalkylphosphoramiden und
- Hexaalkylguanidiniumsalzen und ihren Hydrochloriden.

8. Verfahren nach Anspruch 7, dadurch gekennzeichnet, daß der Katalysator in einer Menge von $10^{-4}$ bis $10^{-1}$ Äquivalent pro Äquivalent umzuwandelnder Säurefunktion verwendet wird.

9. Verfahren nach einem der vorhergehenden Ansprüche, dadurch gekennzeichnet, daß die Temperatur bei Abwesenheit eines Katalysators 110 bis 160°C und bei Verwendung eines Katalysators 80 bis 150°C beträgt.

10. Verfahren nach Anspruch 9, dadurch gekennzeichnet, daß die Temperatur bei Abwesenheit eines Katalysators 120 bis 160°C und bei Verwendung eines Katalysators 100 bis 130°C beträgt.